# EUROPEAN PATENT APPLICATION

(11) **EP 4 438 062 A1**
(43) Date of publication of application: **02.10.2024**
(21) Application number: 22898669.1
(22) Date of filing: 25.11.2022
(51) Int. Cl.: A61K 49/00, A61K 9/19, A61K 31/724, A61K 47/02, A61K 47/12, A61K 47/22, A61K 47/26, A61K 47/69

(54) **INDOCYANINE COMPOUND-CONTAINING SOLID PHARMACEUTICAL COMPOSITION**

(30) Priority: 26.11.2021 JP 2021192251
(71) Applicant: Astellas Pharma Inc., Chuo-ku Tokyo 103-8411 (JP)
(72) Inventor: IKEUCHI, Satomi, Tokyo 103-8411 (JP); YAMAMOTO, Ayano, Tokyo 103-8411 (JP); KATAYAMA, Hiroko, Tokyo 103-8411 (JP); NAKAYA, Hiroko, Tokyo 103-8411 (JP); MURAMATSU, Sumie, Tokyo 103-8411 (JP)
(74) Representative: Hoffmann Eitle
(86) International application number: PCT/JP2022/043595
(87) International publication number: WO 2023/095887

(57) **Abstract**

Provided is a stable solid pharmaceutical composition containing pudexacianinium. The solid pharmaceutical composition includes pudexacianinium or a pharmaceutically acceptable salt thereof, a buffer, and an excipient, and the water content in the solid pharmaceutical composition is 4% by weight or less.

## Description

### TECHNICAL FIELD

The present invention relates to a stable solid pharmaceutical composition containing pudexacianinium.

### BACKGROUND ART

Pudexacianinium is a cyclodextrin-conjugated indocyanine green compound, which is also referred to as TK-1 (NPL 1). The compound is a near-infrared fluorochrome used for fluorescent imaging, includes two cyclodextrin molecules in its structure, and thereby exhibits high hydrophilicity. Due to this high hydrophilicity, the compound causes renal excretion clearance and ureter visualization to be altered compared to an indocyanine green which accumulates in the liver, and is used, for example, as a ureteral imaging agent (NPL 2).

Regarding indocyanine green, NPL 3 is a package insert of a fluorescent angiographic agent with the trade name "Diagnogreen (registered trademark) 25 mg for injection" containing 25 mg of indocyanine green per vial as a lyophilizate (attached solution is 10 mL of water for injection listed in the Pharmacopoeia of Japan). NPL 3 discloses that indocyanine green contains 5.0% or less of sodium iodide, but no disclosure regarding other pharmaceutical additives is provided.

NPL 1 discloses an indocyanine green-containing liquid formulation that contains 9.3% by weight of sucrose and a phosphate buffer solution at pH 7 as an imaging composition for animal administration. PTL 1 exemplifies a liquid and a powder as dosage forms of the imaging composition, and discloses that the powder can be prepared by any suitable known method, for example, by lyophilization or spray drying.

NPL 4 discloses a formulation containing 9.3% by weight of sucrose and PBS at pH 7 as a liposomal indocyanine green formulation for ureteral imaging.

NPL 1 and NPL 2 describe the evaluation of pudexacianinium as an aqueous solution or PBS(phosphate-buffered saline) solution at pH 7.4 for the indocyanine green derivative pudexacianinium or an analogous compound thereof, but do not disclose any pharmaceutical additives.

Further, PTL 2 discloses a diagnostic composition that is separately observed when two or more compounds having significantly different wavelengths of excitation light and/or the fluorescent light are used, and exemplifies, in the description of pharmaceutical additives usually used for formulating, "lactose, starch, sorbitol, D-mannitol, white sugar and the like" as the excipient, and "phosphate, citrate, acetate and the like" as the buffer. PTL 2 does not mention pH for the diagnostic composition, and exemplifies as dosage forms "tablets, powders, fine granules, granules, capsules, syrups, injections, external preparations, and suppositories".

### CITATION LIST

### PATENT LITERATURE

PTL 1: WO2016/128979
PTL 2: Japanese Patent Laid-Open No. 2011-173859

### NON PATENT LITERATURE

NPL 1: Journal of Biomedical Optics 21(8), 086009 (August 2016)
NPL 2: Mol Imaging Biol (2021), published online: 11 May 2021, DOI: 10.1007/s11307-021-01613-0
NPL 3: Diagnogreen (registered trademark) 25 mg for injection; package insert
NPL 4: Nanomedicine: Nanotechnology, Biology and Medicine, Volume 11, Issue 5, July 2015, Pages 1057-1064

### SUMMARY OF INVENTION

### TECHNICAL PROBLEM

Indocyanine green has been already marketed as a lyophilized formulation as disclosed in NPL 3; however, pharmaceutical additives that can sufficiently satisfy the storage stability of the lyophilized formulation has not been examined in a cyclic sugar chain cyclodextrin-binding indocyanine compound including its derivative, pudexacianinium, as far as the present inventors know. PTL 2 describes examples of the excipients and buffers as pharmaceutical additives usually used for formulating; however, there is no disclosure or suggestion from the viewpoint of the storage stability of a lyophilized formulation containing pudexacianinium. The stability of pharmaceutical compositions varies depending on the structures, physical properties, and the like of the compounds, and a technique has been desired to provide a stable solid pharmaceutical composition containing pudexacianinium or a pharmaceutically acceptable salt thereof.

An object of the present invention is to provide a stable solid pharmaceutical composition containing pudexacianinium or a pharmaceutically acceptable salt thereof.

### SOLUTION TO PROBLEM

The present inventors have found that the appearance of related substances of pudexacianinium is suppressed at a certain proportion of water content in a solid pharmaceutical composition of the present invention.

The present invention relates to the following invention:
[1] A solid pharmaceutical composition containing pudexacianinium or a pharmaceutically acceptable salt thereof, a buffer, and an excipient, wherein water content in the solid pharmaceutical composition is 4% by weight or less.
[2] The solid pharmaceutical composition of [1], wherein the solid pharmaceutical composition in the form of an aqueous solution has a pH of 6 or more and 7 or less.
[3] The solid pharmaceutical composition of [1] or [2], wherein the buffer is selected from the group consisting of citrate, phosphate and histidine.
[4] The solid pharmaceutical composition of any of [1] to [3], wherein a buffer concentration is 10 to 50 mmol/L when the solid pharmaceutical composition is in the form of an aqueous solution.
[5] The solid pharmaceutical composition of any of [1] to [4], wherein the excipient is a sugar and/or a salt.
[6] The solid pharmaceutical composition of [5], wherein the sugar is sucrose and/or trehalose.
[7] The solid pharmaceutical composition of [6], wherein a concentration of sucrose and/or trehalose is 5 to 20% (w/v) when the solid pharmaceutical composition is in the form of an aqueous solution.
[8] The solid pharmaceutical composition of [5], wherein the salt is sodium chloride.
[9] The solid pharmaceutical composition of any of [1] to [8], wherein a total amount of related substances after storage under stability test conditions of 40°C and a relative humidity of 75% for 1 month is 7.5% or less.
[10] The solid pharmaceutical composition of any of [1] to [9], wherein the solid pharmaceutical composition is a lyophilized formulation.
[11] The solid pharmaceutical composition of any of [1] to [9], wherein pudexacianinium or a pharmaceutically acceptable salt thereof is pudexacianinium chloride.
[12] A method for producing the solid pharmaceutical composition of [10], comprising(a) preparing a solvent solution containing a solvent, pudexacianinium or a pharmaceutically acceptable salt thereof, a buffer, and an excipient and adjusting a pH of the solvent solution, and (b) lyophilizing the solvent solution with the adjusted pH to obtain a lyophilizate.
[13] A solid pharmaceutical composition containing pudexacianinium or a pharmaceutically acceptable salt thereof, citric acid, and sucrose, wherein water content in the solid pharmaceutical composition is 4% by weight or less, and the solid pharmaceutical composition in the form of an aqueous solution has a pH of 6 or more and 7 or less.

### ADVANTAGEOUS EFFECTS OF INVENTION

According to the present invention, a stable solid pharmaceutical composition containing pudexacianinium can be provided.

### BRIEF DESCRIPTION OF DRAWINGS

[Fig. 1] Fig. 1 shows a graph illustrating quantitative values (%) of pudexacianinium pharmaceutical compositions (lyophilized formulations) having different water contents after storage at 40°C and 75% RH for 3 months and 6 months.
[Fig. 2] Fig. 2 shows a graph illustrating water content (% by weight) of pudexacianinium pharmaceutical compositions (lyophilized formulations) having different water content after storage at 40°C and 75% RH for 1 month, 2 months, and 3 months.
[Fig. 3] Fig. 3 shows a graph illustrating residual rates (%) of the pudexacianinium peak of pudexacianinium pharmaceutical compositions (liquids) having different pH after storage at 70°C for 1 day.
[Fig. 4] Fig. 4 shows a graph illustrating residual rates (%) of the pudexacianinium peak of pudexacianinium pharmaceutical compositions (liquids, pH 6.5) having different buffers after storage at 70°C for 1 day.
[Fig. 5] Fig. 5 shows a graph illustrating peak area ratios (%) of pudexacianinium pharmaceutical compositions (liquids, pH 6.5) having different buffers and excipients after storage at -20°C for 6 months.
[Fig. 6] Fig. 6 shows a graph illustrating the total amounts of related substances (%) of pudexacianinium pharmaceutical compositions (lyophilized formulations) having different excipients (stabilizer) after storage at 40°C and 75% RH for 1 month.
[Fig. 7] Fig. 7 shows a graph illustrating the total amounts of related substances (%) of pudexacianinium pharmaceutical compositions (lyophilized formulations) having different concentrations of drugs and excipients (stabilizer) after storage at 40°C and 75% RH for 1 month.
[Fig. 8] Fig. 8 shows a graph illustrating water content (% by weight) of pudexacianinium pharmaceutical compositions (lyophilized formulations) having different pHs before storage.
[Fig. 9] Fig. 9 shows a graph illustrating water content (% by weight) of pudexacianinium pharmaceutical compositions (lyophilized formulations) having different buffers before storage.
[Fig. 10] Fig. 10 shows a graph illustrating water content (% by weight) of pudexacianinium pharmaceutical compositions (lyophilized formulations) having different excipients before storage.
[Fig. 11] Fig. 11 shows a graph illustrating the total amounts of related substances (%) of lyophilized formulations and liquids of pudexacianinium pharmaceutical compositions after storage at 40°C and 75% RH for 3 months.
[Fig. 12] Fig. 12 shows a graph illustrating the total amounts of related substances (%) of pudexacianinium pharmaceutical compositions (lyophilized formulations) having different pH after storage at 40°C and 75% RH for 3 months.
[Fig. 13] Fig. 13 shows a graph illustrating the total amounts of related substances (%) of pudexacianinium pharmaceutical compositions (lyophilized formulations) having different buffers after storage at 40°C and 75% RH for 3 months.
[Fig. 14] Fig. 14 shows a graph illustrating the total amounts of related substances (%) of pudexacianinium pharmaceutical compositions (lyophilized formulations) having different excipients after storage at 40°C and 75% RH for 3 months.
[Fig. 15] Fig. 15 shows a graph illustrating the change in height (mm) of lyophilized cake from before storage of pudexacianinium pharmaceutical compositions (lyophilized formulations) having different pHs after storage at 40°C and 75% RH for 3 months.
[Fig. 16] Fig. 16 shows a graph illustrating the change in height (mm) of lyophilized cake from before storage of pudexacianinium pharmaceutical compositions (lyophilized formulations) having different buffers after storage at 40°C and 75% RH for 3 months.
[Fig. 17] Fig. 17 shows a graph illustrating the change in height (mm) of lyophilized cake from before storage of pudexacianinium pharmaceutical compositions (lyophilized formulations) having different excipients after storage at 40°C and 75% RH for 3 months.
[Fig. 18] Fig. 18 shows a graph illustrating the reconstitution time (second) of pudexacianinium pharmaceutical compositions (lyophilized formulations) having different pHs after storage at 40°C and 75% RH for 3 months.
[Fig. 19] Fig. 19 shows a graph illustrating the reconstitution time (second) of pudexacianinium pharmaceutical compositions (lyophilized formulations) having different buffers after storage at 40°C and 75% RH for 3 months.
[Fig. 20] Fig. 20 shows a graph illustrating the reconstitution time (second) of pudexacianinium pharmaceutical compositions (lyophilized formulations) having different excipients after storage at 40°C and 75% RH for 3 months.
[Fig. 21] Fig. 21 shows a graph illustrating the total amounts of related substances (%) of pudexacianinium pharmaceutical compositions (lyophilized formulations) having different drug concentrations after storage at 40°C and 75% RH for 6 months.
[Fig. 22] Fig. 22 shows a graph illustrating the total amounts of related substances (%) of pudexacianinium pharmaceutical compositions (lyophilized formulations) having different drug concentrations after storage at 40°C and 75% RH for 6 months.

### DESCRIPTION OF EMBODIMENTS

The term "stable" used herein means that the storage stability of a solid pharmaceutical composition is high. Here, the term "storage stability" means the stability of pudexacianinium in a solid pharmaceutical composition when the solid pharmaceutical composition is stored in a solid state under certain storage conditions, and the storage stability can be evaluated by changes in amount of pudexacianinium itself, or changes in the total amount of related substances derived from pudexacianinium or in amount of a specific related substance.

For example, after the solid pharmaceutical composition is stored under predetermined conditions for a predetermined period, the storage stability can be evaluated by analyzing changes in amount of pudexacianinium itself, or changes in the total amount of related substances derived from pudexacianinium or in amount of a specific related substance, with an analysis means that can measure the amount of pudexacianinium and/or related substances thereof, for example, with a high performance liquid chromatography method (hereinafter, may be abbreviated as HPLC method).

As the storage conditions, 40°C and a relative humidity of 75% (hereinafter, may be abbreviated as %RH) can be typically selected, and as the storage period, 1 month, 2 months, 3 months or 6 months can be typically selected under the above storage conditions.

As a criterion for evaluating the storage stability, in an embodiment, when the residual rate of pudexacianinium after storage under the storage conditions of 40°C and 75% RH for 6 months is typically 90% or more, and preferably 93% or more, it can be determined as "stable". Alternatively, the total amount of related substances derived from pudexacianinium after storage under the storage conditions of 40°C and 75% RH for 3 months is typically 7% or less, preferably 5% or less, and more preferably 3% or less, it can be determined as "stable".

The solid pharmaceutical composition of the present invention is a solid formulation having a water content of 4% by weight or less. In another embodiment, the water content is 3% by weight or less. Note that the lower limit is determined by a drying method to be used (e.g., lyophilization), and, for example, it is 0.5% by weight, and in another embodiment, it is 0.3% by weight. Note that the above upper and lower limits can be arbitrarily combined as desired, for example, as 0.3% by weight to 4% by weight.

The measurement method of the water content of solid pharmaceutical compositions is not particularly limited, and the measurement can be performed typically using the Karl Fischer coulometric titration method, or with a moisture analyzer (Arizona Instrument LLC or AMETEK Brookfield).

For example, in the Karl Fischer coulometric titration method, water content in a sample can be measured by using AQUAMICRON AX (Mitsubishi Chemical Corporation) and AQUAMICRON CXU (Mitsubishi Chemical Corporation) as anolyte and catholyte for water content measurement, respectively, using ultra-dehydrated methanol as sample dissolving solvent, adding a known amount of ultra-dehydrated methanol in a sealed vial to disperse the sample by ultrasound radiation, and then adding 500 µL of supernatant after centrifugation in a moisture meter (Mitsubishi Chemical Analytech Co., Ltd.).

In a method using a moisture analyzer (Arizona Instrument LLC or AMETEK Brookfield), the water content can be calculated by the following: the weight of a vial containing a sample is measured, the sample is placed in a moisture analyzer and heated to measure water content in the sample, the vial for the sample after measurement is washed and dried, the weight of the empty vial is measured to calculate the weight of the sample, and then the water content is divided by the weight of the sample.

The solid pharmaceutical composition of the present invention in the form of an aqueous solution has a pH of typically 6 to 7, 5 to 8 in another embodiment, and 4 to 9 in yet another embodiment. Note that the above upper and lower limits can be arbitrarily combined as desired, for example, as 6 to 8.

Pudexacianinium used for the solid pharmaceutical composition of the present invention is a compound represented by the following structural formula:

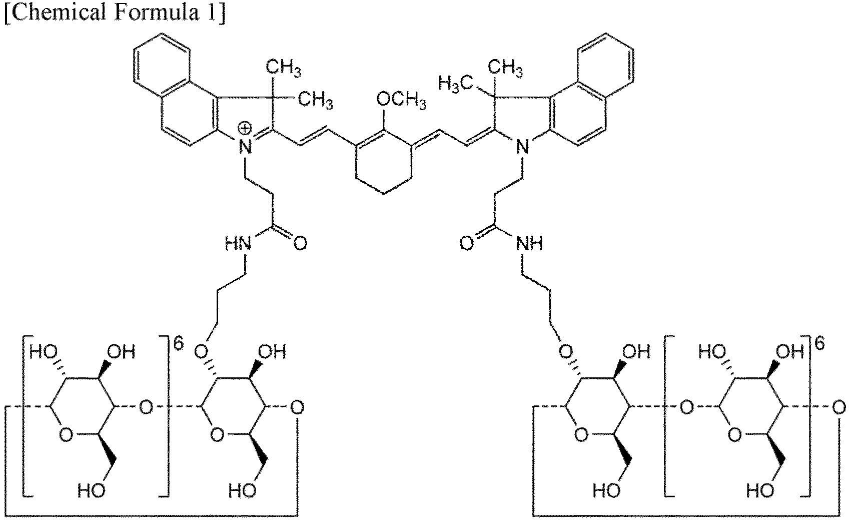

Pudexacianinium or a pharmaceutically acceptable salt thereof is easily available by a production method described in NPL 1, WO2011/093098 or WO2021/105888, or a production method based thereon. Pudexacianinium or a pharmaceutically acceptable salt thereof can be an amorphous body obtained by the production method described in NPL 1. Alternatively, it can be crystalline (WO2021/105888).

In a pharmaceutically acceptable salt of pudexacianinium, pudexacianinium may form an acid addition salt with an acid. Specific examples thereof include acid addition salts with inorganic acids such as hydrochloric acid, hydrobromic acid, hydroiodic acid, sulfuric acid, nitric acid, and phosphoric acid; acid addition salts with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, and glutamic acid; and the like. A pharmaceutically acceptable salt of pudexacianinium is typically an acid addition salt with hydrochloric acid, pudexacianinium chloride.

Pudexacianinium or a pharmaceutically acceptable salt thereof is a near-infrared fluorochrome and useful for near-infrared fluorescent imaging, such as ureteral imaging, lymphatic imaging, and cancer imaging.

The amount of pudexacianinium or a pharmaceutically acceptable salt thereof to be administered can be appropriately determined based on individual cases, for example, in consideration of organs and/or tissues of a subject to be imaged, administration routes, patient's age, race and/or gender.

A single dose is typically 0.3 to 24 mg/adult, in terms of the free form of pudexacianinium.

The blending ratio of pudexacianinium or a pharmaceutically acceptable salt thereof is typically 0.1 to 7% by weight, 0.05 to 16% by weight in another embodiment, and 0.05 to 28% by weight in yet another embodiment with respect to the weight of the solid pharmaceutical composition. Note that the above upper and lower limits can be arbitrarily combined as desired, for example, as 0.1 to 16% by weight.

In addition, the pudexacianinium concentration is typically 0.1 to 8 mg/mL when the solid pharmaceutical composition is in the form of an aqueous solution.

The buffer to be used for the solid pharmaceutical composition of the present invention is not particularly limited as long as the buffer can set the pH of the solid pharmaceutical composition in the form of an aqueous solution of 6 or more and 7 or less. The buffer can be typically selected from the group consisting of citrate, phosphate and histidine, in another embodiment, it is citrate or phosphate, and in yet another embodiment, it is citrate.

The blending ratio of the buffer is typically 3 to 15% by weight, in another embodiment, it is 1 to 25% by weight, and in yet another embodiment, it is 1 to 40% by weight with respect to the weight of the solid pharmaceutical composition. Note that the above upper and lower limits can be arbitrarily combined as desired, for example, as 3 to 25% by weight.

Further, the buffer concentration is typically 10 to 50 mmol/L, in another embodiment, it is 5 to 100 mmol/L, and in yet another embodiment, it is 5 to 200 mmol/L when the solid pharmaceutical composition is in the form of an aqueous solution. Note that the above upper and lower limits can be arbitrarily combined as desired, for example, as 10 to 100 mmol/L.

The excipient to be used for the solid pharmaceutical composition of the present invention is not particularly limited as long as the excipient can retain or improve the storage stability of the solid pharmaceutical composition when stored in a solid state. The excipient is typically a sugar and/or a salt (i.e., a sugar or a salt, or a combination of a sugar and a salt).

When a sugar is used as the excipient, it is typically sucrose and/or trehalose (i.e., sucrose or trehalose, or a combination of sucrose and trehalose), and in another embodiment, it is sucrose.

When a sugar is used as the excipient, the sugar concentration (typically, the concentration of sucrose and/or trehalose) is typically 5 to 20% (w/v), in another embodiment, it is 2 to 30% (w/v), and yet another embodiment, 2 to 50% (w/v) when the solid pharmaceutical composition is in the form of an aqueous solution. Note that the above upper and lower limits can be arbitrarily combined as desired, for example, as 5 to 30% (w/v).

Since if the excipient concentration is too high, the reconstitution time becomes longer, and if the concentration is too low, the stability becomes lower, it is good to select an adequate concentration.

When a salt is used as the excipient, it is typically sodium chloride.

When a salt is used as the excipient, the salt concentration (typically, the sodium chloride concentration) is typically 0.9 to 1.8% (w/v), in another embodiment, it is 0.5 to 2.7% (w/v), and yet another embodiment, 0.5 to 4.5% (w/v) when the solid pharmaceutical composition is in the form of an aqueous solution. Note that the above upper and lower limits can be arbitrarily combined as desired, for example, as 0.9 to 2.7% (w/v).

The dosage form of the solid pharmaceutical composition of the present invention is not particularly limited as long as it is a solid pharmaceutical composition that can be dissolved at the time of use, and the examples thereof can include a lyophilized formulation, spray-dried formulation, and the like. The lyophilized formulation is preferable from the viewpoint of its excellent stability.

The solid pharmaceutical composition of the present invention can be formulated, if desired, by appropriately using various pharmaceutical additives in addition, within the extent that the desired effects described herein can be achieved. Such pharmaceutical additives are not particularly limited as long as they are pharmaceutically and pharmacologically acceptable, and excipients, antioxidants, surfactants, or the like can be used. Various pharmaceutical additives can be appropriately used in an adequate amount, within an amount range where the desired effects of the present invention can be achieved.

Examples of the excipient include lactose, starch, sorbitol, D-mannitol, white sugar, and the like.

Examples of the antioxidant include methionine, erythorbic acid, dibutylhydroxytoluene, butylhydroxyanisole, α-tocopherol, tocopherol acetate, L-ascorbic acid and a salt thereof, L-ascorbic acid palmitate, L-ascorbic acid stearate, sodium bisulfite, sodium sulfite, triamyl gallate, propyl gallate, or chelating agents such as disodium ethylenediaminetetraacetic acid (EDTA), sodium pyrophosphate, and sodium metaphosphate.

Examples of the surfactant include, specifically, for example, nonionic surfactants, for example, sorbitan fatty acid esters such as sorbitan monocaprylate, sorbitan monolaurate and sorbitan monopalmitate; glycerin fatty acid esters such as glycerol monocaprylate, glycerol monomyristate and glycerol monostearate; polyglycerol fatty acid esters such as decaglyceryl monostearate, decaglyceryl distearate and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate and polyoxyethylene sorbitan tristearate; polyoxyethylene sorbitol fatty acid esters such as polyoxyethylene sorbitol tetrastearate and polyoxyethylene sorbitol tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether and polyoxyethylene polyoxypropylene cetyl ether; polyoxyethylene alkylphenyl ethers such as polyoxyethylene nonylphenyl ether; polyoxyethylene hardened castor oils such as polyoxyethylene castor oil and polyoxyethylene hydrogenated castor oil; polyoxyethylene beeswax derivatives such as polyoxyethylene sorbitol beeswax; polyoxyethylene lanolin derivatives such as polyoxyethylene lanolin; and surfactants having an HLB of 6 to 18 such as polyoxyethylene fatty acid amides, e.g., polyoxyethylene octadecanamide; and anionic surfactants, for example, alkyl sulfates having a C₁₀-C₁₈ alkyl group such as sodium cetyl sulfate, sodium lauryl sulfate and sodium oleyl sulfate; polyoxyethylene alkyl ether sulfates with average numbers of moles of added ethylene oxide units of 2 to 4 and having 10 to 18 carbon atoms of alkyl group such as sodium polyoxyethylene lauryl sulfate; alkyl sulfosuccinate salts having C₈-C₁₈ alkyl group such as sodium lauryl sulfosuccinate; natural surfactants such as lecithin and glycerophospholipid; sphingophospholipids such as sphingomyelin; or sucrose esters of C₁₂-C₁₈ fatty acid.

Since the solid pharmaceutical composition of the present invention is a solid formulation, such a formulation can be used as an injection, for example, for near-infrared fluorescent imaging, by adding an appropriate solvent for injection (typically water, and particularly, sterile water) thereto before use.

The amount of the solvent for injection to be added for making the solid pharmaceutical composition an injection (e.g., mL) is typically 0.25 to 1-fold (vol/wt), in another embodiment, 0.1 to 1.5-fold (vol/wt), and in still another embodiment, 0.1 to 5-fold (vol/wt), and yet still another embodiment 0.1 to 8-fold (vol/wt), respective to the weight of the solid pharmaceutical composition (e.g., mg). Note that the above upper and lower limits can be arbitrarily combined as desired, for example, as 0.25 to 1.5-fold (vol/wt).

Hereinafter, the production method of the present invention regarding a lyophilized formulation, which is an embodiment of the solid pharmaceutical composition of the present invention, will be described; however, the present invention is not limited thereto.

The production method of the present invention includes:
(a) preparing a solvent solution containing a solvent, pudexacianinium or a pharmaceutically acceptable salt thereof, a buffer, and an excipient and adjusting a pH of the solvent solution; and
(b) lyophilizing the solvent solution with the pH adjusted to obtain a lyophilizate.

The description for the solid pharmaceutical composition of the present invention can apply as it is to the "pudexacianinium or a pharmaceutically acceptable salt thereof", the "buffer", the "excipient" and the like used in the "production method" of the present invention.

In step (a) of the production method of the present invention, a pudexacianinium or a pharmaceutically acceptable salt thereof, a buffer, and an excipient are prepared, and a solvent that can dissolve all of these is also prepared. Examples of the solvent can include water.

A solvent solution is prepared by mixing a solvent, pudexacianinium or a pharmaceutically acceptable salt thereof, a buffer, and an excipient and dissolving them by shaking or the like, and then the pH of the solvent solution is adjusted typically to 6 to 7, in another embodiment, to 5 to 8, and in yet another embodiment, to 4 to 9. Note that the above upper and lower limits can be arbitrarily combined as desired, for example, as 6 to 8.

In step (b) of the production method of the present invention, a lyophilized formulation, which is an embodiment of the solid pharmaceutical composition of the present invention, can be obtained as a lyophilizate, by lyophilizing the solvent solution of which pH has been adjusted to a predetermined pH. The lyophilization can be conducted by a conventional method.

### EXAMPLES

The present invention will now be more specifically described with reference to Examples; however, the present invention is not limited thereto.

### [Example 1]

In 20 mmol/L of citrate buffer solution (pH 6.5) containing 10% (w/v) sucrose, pudexacianinium was dissolved so as to be 1 mg/mL in terms of the free form to obtain an aqueous solution of pudexacianinium. After confirming the pH of the solution, it was adjusted as needed, filled into a vial, and lyophilized to obtain a pudexacianinium pharmaceutical composition (lyophilized formulation) having water content of 1.3%. The vial was repressurized with nitrogen, capped with a rubber stopper, and crimped with an aluminum cap.

### [Example 2]

A pudexacianinium pharmaceutical composition (lyophilized formulation) having water content of 3.0% by weight was prepared by opening the rubber stopper of the pudexacianinium lyophilized formulation prepared in Example 1 in a constant temperature and humidity chamber at 25°C and 40% RH, and adjusting the storage time.

### [Comparative Example 1]

A pudexacianinium pharmaceutical composition (lyophilized formulation) was prepared in the same manner as in Example 2, except that the water content was changed to 4.2% by weight.

### [Test Example 1] Stability Test

A storage stability test (stored at 40°C and 75% RH for 6 months) was conducted on each sample to evaluate the stability of pharmaceutical compositions (lyophilized formulations). The quantitative values of pudexacianinium before and after storage were then evaluated by a high performance liquid chromatography (HPLC). The test method is as follows.

To an HPLC system, a CAPCELL CORE AQ column (OSAKA SODA CO., LTD.) was connected, 10 mmol/L of phosphate buffer solution, pH 7.3 and acetonitrile were connected to a mobile phase A line and a mobile phase B line, respectively, to flow at a flow rate of 1.0 mL/min. The samples were diluted to 0.26 mg/mL with the mobile phase A, and 5 µL thereof was injected. The gradient program shown in Table 1 was applied. The detection was performed at UV 255 nm. The column temperature and the sample temperature were set to 45°C and 5°C, respectively.

**[Table 1]**

| Duration after injection (min) | Mobile phase A (%) | Mobile phase B (%) |
|---|---|---|
| 0.0→10.0 | 75→63 | 25→37 |
| 10.0→10.1 | 63→15 | 37→85 |
| 10.1→13.0 | 15 | 85 |
| 13.0→13.1 | 15->75 | 85→25 |
| 13.1→17.0 | 75 | 25 |

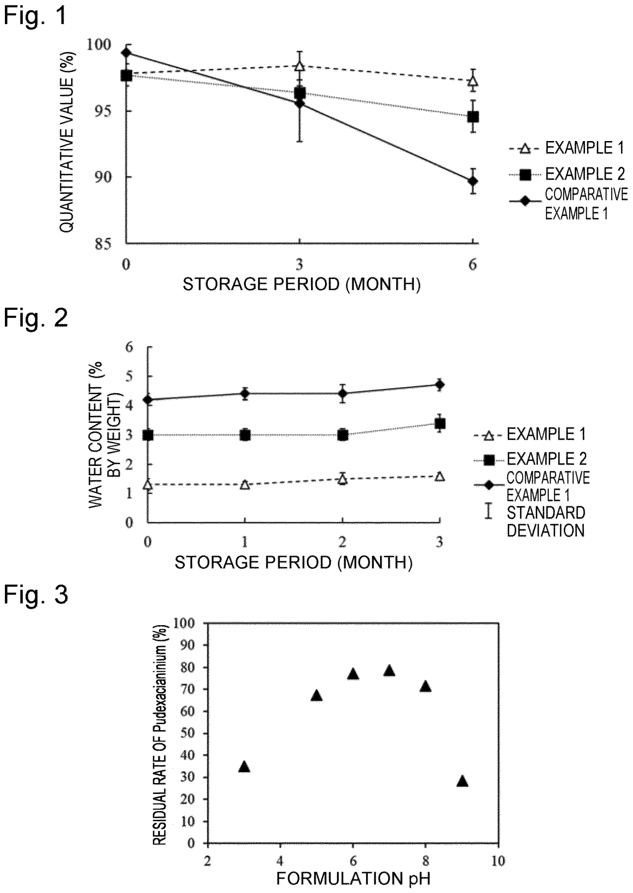

For quantitative value measurement, a peak area of pudexacianinium detected by HPLC was measured by an automated analysis method, and the quantitative values (%) were calculated by an external standard method. The evaluation results of the quantitative values are shown in Fig. 1.

### [Test Example 2] Water Content Measurement

A storage stability test (stored at 40°C and 75% RH for 6 months) was conducted on each sample to evaluate the stability of pudexacianinium pharmaceutical compositions (lyophilized formulations). The water content measurement of pudexacianinium pharmaceutical compositions (lyophilized formulations) before and after storage was then evaluated. The test method is as follows.

The Karl Fischer coulometric titration method was used to measure the water content of the lyophilized formulations of Example 1, Example 2, and Comparative Example 1. AQUAMICRON AX (Mitsubishi Chemical Corporation) and AQUAMICRON CXU (Mitsubishi Chemical Corporation) were used as anolyte and catholyte for water content measurement, respectively, and ultra-dehydrated methanol was used as sample dissolving solvent. A known amount of ultra-dehydrated methanol was added in a sealed vial to disperse lyophilized cake by ultrasound radiation. 500 µL of supernatant after centrifugation was added to a moisture meter (Mitsubishi Chemical Analytech Co., Ltd.) to measure the water content in the samples. The measurement results of water content are shown in FIG. 2.

Fig. 1 shows that the quantitative value after storage in Comparative Example 1 was less than 90%, and therefore, the lyophilized formulation was stable when its watercontent was 4% or less. On the other hand, referring to Fig. 2, almost no increase in water content was observed with storage, and it was thus confirmed that the water content in the lyophilized formulation immediately after the production contributes to the stability.

### [Reference Example 1] <pH> Sample Preparation

100 mmol/L of phosphate buffer solution (pH 6) was used to dissolve pudexacianinium so that the pudexacianinium concentration is 1 mg/mL, to prepare a composition of Reference Example 1. The formulation is as shown in Table 2 below.

### [Reference Example 2] <pH> Sample Preparation

A composition of Reference Example 2 was prepared by using the same method as in Reference Example 1 except that pH was set to 7. The formulation is as shown in Table 2 below.

### [Reference Example 3] <pH> Sample Preparation

A composition of Reference Example 3 was prepared by using the same method as in Reference Example 1 except that pH was set to 3. The formulation is as shown in Table 2 below.

### [Reference Example 4] <pH> Sample Preparation

A composition of Reference Example 4 was prepared by using the same method as in Reference Example 1 except that pH was set to 5. The formulation is as shown in Table 2 below.

### [Reference Example 5] <pH> Sample Preparation

A composition of Reference Example 5 was prepared by using the same method as in Reference Example 1 except that pH was set to 8. The formulation is as shown in Table 2 below.

### [Reference Example 6] <pH> Sample Preparation

100 mmol/L of carbonate buffer solution (pH 9) was used to dissolve pudexacianinium so that the pudexacianinium concentration is 1 mg/mL, to prepare a composition of Reference Example 6. The formulation is as shown in Table 2 below.

**[Table 2]**

| Ingredient | Reference example 1 | Reference example 2 |
|---|---|---|
| Pudexacianinium | 50 mg | 50 mg |
| Sodium chloride | 0.485 g | 0.194 g |
| Sodium dihydrogen phosphate dihydrate | 0.649 g | 0.259 g |
| Disodium hydrogen phosphate 12-hydrate | 0.301 g | 1.196 g |
| Water | q. s. to 50 mL | q. s. to 50 mL |
| pH | 6 | 7 |

| Ingredient | Reference example 3 | Reference example 4 | Reference example 5 | Reference example 6 |
|---|---|---|---|---|
| Pudexacianinium | 50 mg | 50 mg | 50 mg | 50 mg |
| Sodium chloride | 0.611 g | 0.574 g | 0.029 g | 0.497 g |
| Sodium dihydrogen phosphate dihydrate | 0.710 g | 0.764 g | 0.037 g | - |
| Disodium hydrogen phosphate 12-hydrate | - | 0.036 g | 1706 g | - |
| 0.1 mol/L of phosphate | 4.5 mL | - | - | - |
| Sodium bicarbonate | - | - | - | 0.356 g |
| Sodium carbonate | - | - | - | 0.081 g |
| Water | q. s. to 50 mL | q. s. to 50 mL | q. s. to 50 mL | q.s.to 50 mL |
| pH | 3 | 5 | 8 | 9 |

### [Reference Test Example 1]

A heat stress test (stored at 70°C for 1 day) was conducted on the compositions described in Reference Examples 1 to 6 to evaluate the stability of liquids. The quantitative values of pudexacianinium before and after the heat stress test were then evaluated by a high performance liquid chromatography (HPLC). Analysis conditions are as follows.

To the HPLC system, a Meteoric Core C18 BIO column (YMC CO., LTD.) was connected, 100 mmol/L of aqueous ammonium acetate solution and acetonitrile were connected to the mobile phase A line and the mobile phase B line, respectively, to flow at a flow rate of 0.3 mL/min. The samples were diluted to 0.1 mg/mL with 50% (v/v) aqueous methanol solution, and 50 µL thereof was injected. The gradient program shown in Table 3 was applied. The detection was performed at UV 254 nm. The column temperature and the sample temperature were set to 50°C and 5°C, respectively.

**[Table 3]**

| Gradient program | | |
|---|---|---|
| Duration after injection(min) | Mobile phase A (%) | Mobile phase B (%) |
| 0 | 99 | 1 |
| 2 | 80 | 20 |
| 35 | 70 | 30 |
| 45 | 10 | 90 |
| 50 | 10 | 90 |
| 50.1 | 99 | 1 |
| 60 | 99 | 1 |

A peak area of pudexacianinium detected by HPLC was measured by an automated analysis method, and the quantitative values (mg/mL) were calculated by an external standard method. The quantitative values after storage were divided by the quantitative values before storage to obtain residual rates of pudexacianinium peak(%).

The evaluation results of the quantitative values obtained in Reference Examples are shown in Fig. 3. The results confirmed that being at pH 6 or pH 7 satisfied the stability. On the other hand, the quantitative values at pH 3 to 5 and pH 8 to 9 significantly lowered with the heat stress test, and therefore, around pH 6 to 7 was confirmed to be the optimal pH.

### [Reference Example 7] <Type and Concentration of Buffer>

10 to 50 mmol/L of phosphate buffer solution (pH 6.5), 10 to 50 mmol/L of citrate buffer solution (pH 6.5), and 10 to 50 mmol/L of histidine buffer solution (pH 6.5) were used to dissolve pudexacianinium so that the pudexacianinium concentration is 1 mg/mL, to prepare evaluation samples of sample Nos. A1 to A9. The formulation of each evaluation sample is as shown in Table 4 below.

**[Table 4]**

| Ingredient | A1 | A2 | A3 | A4 | A5 | A6 | A7 | A8 | A9 |
|---|---|---|---|---|---|---|---|---|---|
| Pudexacianinium (mg/mL) | 1 | | | | | | | | |
| NaCl (mg/mL) | 9 | | | | | | | | |
| Phosphate (mmol/L) | 10 | 20 | 50 | - | - | - | - | - | - |
| Citrate (mmol/L) | - | - | - | 10 | 20 | 50 | - | - | - |
| Histidine (mmol/L) | - | - | - | - | - | - | 10 | 20 | 50 |
| NaOH/HCl | Adjusted to pH 6.5 | | | | | | | | |

### [Reference Test Example 2] <Type and Concentration of Buffer>

A heat stress test (stored at 70°C for 1 day) was conducted to evaluate the stability of liquids. The quantitative values of pudexacianinium before and after the heat stress test were then evaluated by a high performance liquid chromatography (HPLC). The analysis conditions are the same as Reference Test Example 1.

A peak area of pudexacianinium detected by HPLC was measured by an automated analysis method, and the quantitative values (mg/mL) were calculated by an external standard method. The quantitative values after storage were divided by the quantitative values before storage to obtain residual rates of pudexacianinium peak(%).

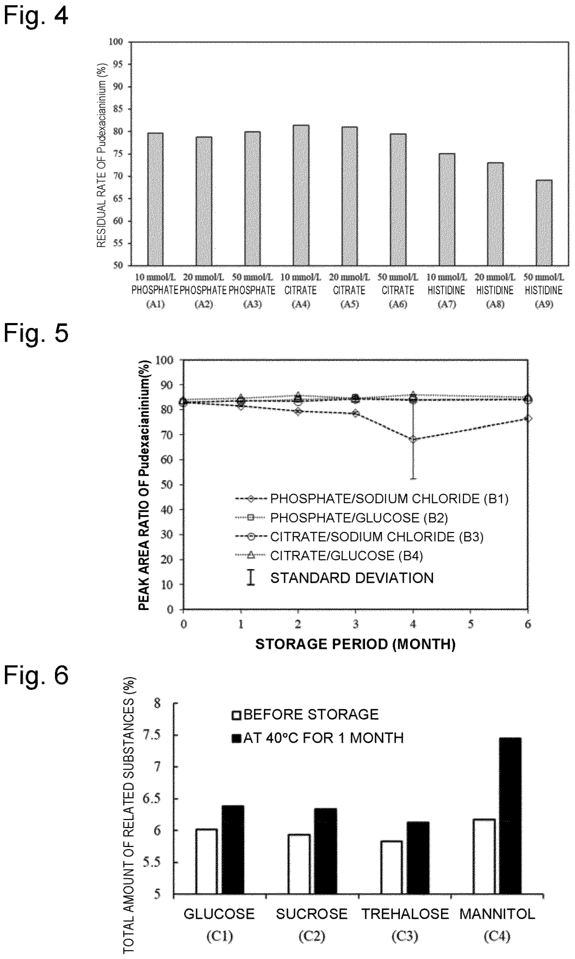

The evaluation results of the quantitative values obtained in this Reference Example are shown in Fig. 4. The results confirmed that the concentration range of 10 to 50 mmol/L satisfied the stability, when it comes to phosphate or citrate buffer solution. On the other hand, the results showed that the stability of histidine buffer solution was inferior to that of phosphate or citrate buffer solution. From the above results, it was confirmed to be stable in 10 to 50 mmol/L of phosphate buffer solution or citrate buffer solution at pH 6.5.

### [Reference Example 8] <Type of Buffer and Selection of Excipient>

20 mmol/L of phosphate buffer solution (pH 6.5) or 20 mmol/L of citrate buffer solution (pH 6.5) both of which contained 0.9% (w/v) sodium chloride or 5% (w/v) glucose as the excipient was used to dissolve pudexacianinium so that the pudexacianinium concentration is 4 mg/mL, to prepare evaluation samples of sample Nos. B 1 to B4. The formulation of each evaluation sample is as shown in Table 5 below.

**[Table 5]**

| Sample No. | Pudexacianinium concentration | Buffer | pH | Tonicity agent |
|---|---|---|---|---|
| B-1 | 4 mg/mL | 20 mmol/L of phosphate | 6.5 | 0.9% (w/v) of sodium chloride |
| B-2 | | | | 5% (w/v) of glucose |
| B-3 | | 20 mmol/L of citrate | | 0.9% (w/v) of sodium chloride |
| B-4 | | | | 5% (w/v) of glucose |

### [Reference Test Example 3] <Type of Buffer and Selection of Excipient>

A storage stability test (stored at -20°C for 6 months) was conducted on each sample to evaluate the stability of liquids. The peak ratios (%) of pudexacianinium before and after the storage stability test were then evaluated by a high performance liquid chromatography (HPLC). The analysis conditions are the same as Reference Test Example 1.

A peak area of pudexacianinium detected by HPLC was measured by an automated analysis method, and the peak area ratios (%) of pudexacianinium to the total peaks detected were calculated to evaluate the quantitative values.

The evaluation results of the storage stability obtained in this Reference Example are shown in Fig. 5. In the combination of phosphate buffer solution and sodium chloride, it was observed that the quantitative values during storage tended to decrease over time. On the other hand, the decrease in quantitative values during storage was not observed in combinations of phosphate buffer solution and glucose, citrate buffer solution and sodium chloride, and citrate buffer solution and glucose. In addition, the citrate buffer solution was suggested to be desirable among others.

### [Examples 3] <Excipient (Stabilizer)>

20 mmol/L each of citrate buffer solution (pH 6.5) that contained 5% (w/v) glucose or 10% (w/v) sucrose or 10% (w/v) trehalose or 5% (w/v) mannitol was used to dissolve pudexacianinium so that the pudexacianinium concentration is 4 mg/mL, to prepare evaluation sample aqueous solution of sample Nos. C1 to C4. The formulation is as shown in Table 6 below. Each evaluation sample aqueous solution was filled into a vial, and lyophilized to obtain a pudexacianinium pharmaceutical composition (lyophilized formulation). The vial was repressurized with nitrogen, capped with a rubber stopper, and crimped with an aluminum cap.

**[Table 6]**

| Sample No. | Pudexacianinium concentration | Buffer/pH | Tonicity agent |
|---|---|---|---|
| C-1 | 4 mg/mL | 20 mmol/L of citrate pH 6.5 | 5% (w/v) of glucose |
| C-2 | | | 10% (w/v) of sucrose |
| C-3 | | | 10% (w/v) of trehalose |
| C-4 | | | 5% (w/v) of mannitol |

### [Test Example 3] Appearance Evaluation

It was observed that the appearance of the lyophilized formulation obtained in this Example had shrinkage and porosity of the lyophilized cake when glucose was selected as the excipient. The other excipients exhibited a good appearance and reconstitution.

### [Test Example 4]

A storage stability test (stored at 40°C and 75% RH for 1 month) was conducted on each sample to evaluate the stability of pharmaceutical compositions (lyophilized formulations). The amounts of related substances of pudexacianinium before and after storage were then evaluated by a high performance liquid chromatography (HPLC). Analysis conditions are as follows.

To the HPLC system, a Kinetex C18 column (Phenomenex, Inc.) was connected, 10 mmol/L of phosphate buffer solution, pH 3.0 and methanol were connected to the mobile phase A line and the mobile phase B line, respectively, to flow at a flow rate of 1.2 mL/min. After samples were redissolved by adding water to have its concentration of 4 mg/mL, these were diluted to 2 mg/mL with a mix solution of the mobile phase A/methanol (4:1), and 80 µL thereof was injected. The gradient program shown in Table 7 was applied. The detection was performed at UV 250 nm. The column temperature and the sample temperature were set to 40°C and 5°C, respectively.

**[Table 7]**

| Duration after injection (min) | Mobile phase A (%) | Mobile phase B (%) |
|---|---|---|
| 0 | 95 | 5 |
| 30 | 65 | 35 |
| 80 | 65 | 35 |
| 100 | 5 | 95 |
| 100.1 | 95 | 5 |
| 110 | 95 | 5 |

Peak areas of the related substances and pudexacianinium detected by HPLC were measured by an automated analysis method, and the peak area ratios (%) of each related substance and pudexacianinium were determined. The total amount of related substances was calculated by subtracting the peak area ratio of the detected pudexacianinium from 100%.

The evaluation results of the storage stability of the lyophilized formulation obtained in this Example are shown in Fig. 6. The total amount of related substances increased the most when mannitol was selected as the excipient. By comprehensive judgment, it was confirmed that stable lyophilized formulation can be obtained by selecting sucrose or trehalose as the excipient.

### [Examples 4] <Effect of Concentration of Excipient (Stabilizer)> Method of Preparing Samples

In this study, to evaluate the effect of the type and concentration of excipient, 20 mmol/L each of citrate buffer solution (pH 6.5) that contained 10% (w/v) sucrose or 10% (w/v) trehalose was used to dissolve pudexacianinium so that the pudexacianinium concentration is 0.45 mg/mL, to prepare evaluation sample aqueous solution of sample Nos. D1 and D2. Similarly, 40 mmol/L each of citrate buffer solution (pH 6.5) that contained 20% (w/v) sucrose or 20% (w/v) trehalose was used to dissolve pudexacianinium so that the pudexacianinium concentration is 0.9 mg/mL, to prepare evaluation sample aqueous solution of sample Nos. D3 and D4. The formulation of each evaluation sample aqueous solution is as shown in Table 8 below. Each evaluation sample aqueous solution was filled into a vial, and lyophilized to obtain a pudexacianinium pharmaceutical composition (lyophilized formulation). The vial was repressurized with nitrogen, capped with a rubber stopper, and crimped with an aluminum cap.

**[Table 8]**

| Sample No. | Pudexacianinium concentration | Buffer/pH | Tonicity agent |
|---|---|---|---|
| D1 | 0.45 mg/mL | 20 mmol/L of citrate pH 6.5 | 10% (w/v) of sucrose |
| D2 | | | 10 (w/v)% of trehalose |
| D3 | 0.9 mg/mL | 40 mmol/L of citrate pH 6.5 | 20 (w/v)% of sucrose |
| D4 | | | 20 (w/v)% of trehalose |

### [Test Example 5] Stability Evaluation

A storage stability test (stored at 40°C and 75% RH for 1 month) was conducted on each sample to evaluate the stability of lyophilized formulation. The amounts of related substances of pudexacianinium before and after storage were then evaluated by a high performance liquid chromatography (HPLC). Analysis conditions are as follows.

The same procedure was conducted as in Test Example 4 except that samples were redissolved with water to have their pudexacianinium concentrations of 0.45 mg/mL, and 80 µL thereof was injected.

Peak areas of the related substances and pudexacianinium detected by HPLC were measured by an automated analysis method, and the peak area ratios (%) of each related substance and pudexacianinium were determined. The total amount of related substances was calculated by subtracting the peak area ratio of the detected pudexacianinium from 100%.

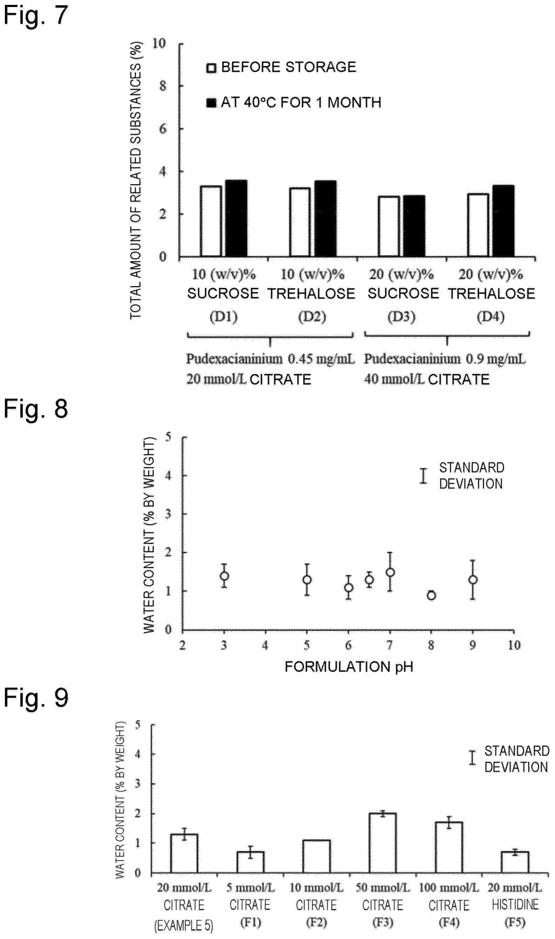

The evaluation results of the storage stability obtained in this Example are shown in Fig. 7. When sucrose and trehalose were selected as the excipient, the increased amounts of related substances after storage were almost the same in each case. From the above results, it was confirmed that a stable lyophilized formulation can be obtained in the formulation of 20 to 40 mmol/L of citrate buffer solution containing 10 to 20% (w/v) sucrose or trehalose.

### [Example 5]

In 20 mmol/L of citrate buffer solution (pH 6.5) containing 10% (w/v) sucrose, pudexacianinium was dissolved so as to be 1 mg/mL in terms of the free form to obtain an aqueous solution of pudexacianinium. After confirming the pH of the solution, it was filled into a vial, and lyophilized to obtain a pudexacianinium pharmaceutical composition (lyophilized formulation). The vial was repressurized with nitrogen, capped with a rubber stopper, and crimped with an aluminum cap.

### [Comparative Example 2]

The drug product solution prepared in the same manner as in Example 5 was filled into a vial, capped with a rubber stopper, and crimped with an aluminum cap.

### [Example 6]

The same method as in Example 5 was used to prepare evaluation samples of sample Nos. E1 to E6, except that 20 mmol/L of phosphate buffer solution (pH 3 to 8) or carbonate-bicarbonate buffer solution (pH 9) was used as the buffer solution. The formulation of each evaluation sample is as shown in Table 9 below.

**[Table 9]**

| Sample No. | Pudexacianinium concentration | Buffer | pH | Excipient |
|---|---|---|---|---|
| E1 | 1 mg/mL | 20 mol/L of phosphate buffer solution | 3 | 10% (w/v) of sucrose |
| E2 | | | 5 | |
| E3 | | | 6 | |
| E4 | | | 7 | |
| E5 | | | 8 | |
| E6 | | 20 mol/L of carbonate-bicarbonate buffer solution | 9 | |

### [Example 7]

The same method as in Example 5 was used to prepare evaluation samples of sample Nos. F1 to F5, except that 5 to 100 mmol/L of citrate buffer solution (pH 6.5) or 20 mmol/L of histidine buffer solution (pH 6.5) was used as the buffer solution. The formulation of each evaluation sample is as shown in Table 10 below.

**[Table 10]**

| Sample No. | Pudexacianinium concentration | Buffer | | pH | Excipient |
|---|---|---|---|---|---|
| F1 | 1 mg/mL | 5 mmol/L | Citrate buffer solution | 6.5 | 10% (w/v) of sucrose |
| F2 | | 10 mmol/L | | | |
| F3 | | 50 mmol/L | | | |
| F4 | | 100 mmol/L | | | |
| F5 | | 20 mmol/L | Histidine buffer solution | | |

### [Example 8]

The same method as in Example 5 was used to prepare evaluation samples of sample Nos. G1 to G4, except that 5 to 40% (w/v) sucrose or 10% (w/v) trehalose was used as the excipient. The formulation of each evaluation sample is as shown in Table 11 below.

**[Table 11]**

| Sample No. | Pudexacianinium concentration | Buffer | pH | Excipient | |
|---|---|---|---|---|---|
| G1 | 1 mg/mL | 20 mmol/L of citrate buffer solution | 6.5 | 5 (w/v)% | Sucrose |
| G2 | | | | 20 (w/v)% | |
| G3 | | | | 40 (w/v)% | |
| G4 | | | | 10 (w/v)% | Trehalose |

### [Test Example 6]

The water content measurement of the prepared pharmaceutical compositions (lyophilized formulations) before storage were evaluated. The test method was performed in the same manner as in Test Example 2.

The measurement results of the water content before storage obtained in Examples 5 to 8 are shown in Figs. 8 to 10.

### [Test Example 7]

A storage stability test (stored at 40°C and 75% RH for 3 months) was conducted on each sample to evaluate the stability of pharmaceutical compositions (lyophilized formulations and liquids). The amounts of related substances of pudexacianinium before and after storage were then evaluated by a high performance liquid chromatography (HPLC). The test method is as follows.

To the HPLC system, a CAPCELL CORE AQ column (OSAKA SODA CO., LTD.) was connected, 10 mmol/L of phosphate buffer solution, pH 7.3 and acetonitrile were connected to the mobile phase A line and the mobile phase B line, respectively, to flow at a flow rate of 1.0 mL/min. The samples were diluted to 0.52 mg/mL with the mobile phase A, and 20 µL thereof was injected. The gradient program shown in Table 12 was applied. The detection was performed at UV 255 nm. The column temperature and the sample temperature were set to 45°C and 5°C, respectively.

**[Table 12]**

| Duration after injection (min) | Mobile phase A (%) | Mobile phase B (%) |
|---|---|---|
| 0.0→18.0 | 95→81 | 5→19 |
| 18.0→54.0 | 81→67 | 19-→33 |
| 54.0→59.0 | 67→30 | 33→70 |
| 59.0→70.0 | 30 | 70 |
| 70.0→70.1 | 30→95 | 70→5 |
| 70.1→80 | 95 | 5 |

Peak areas of the related substances and pudexacianinium detected by HPLC were measured by an automated analysis method, and the amount of each related substance (%) were determined by an external standard method. The total amount of related substances was determined by the total sum of the amount of each related substance.

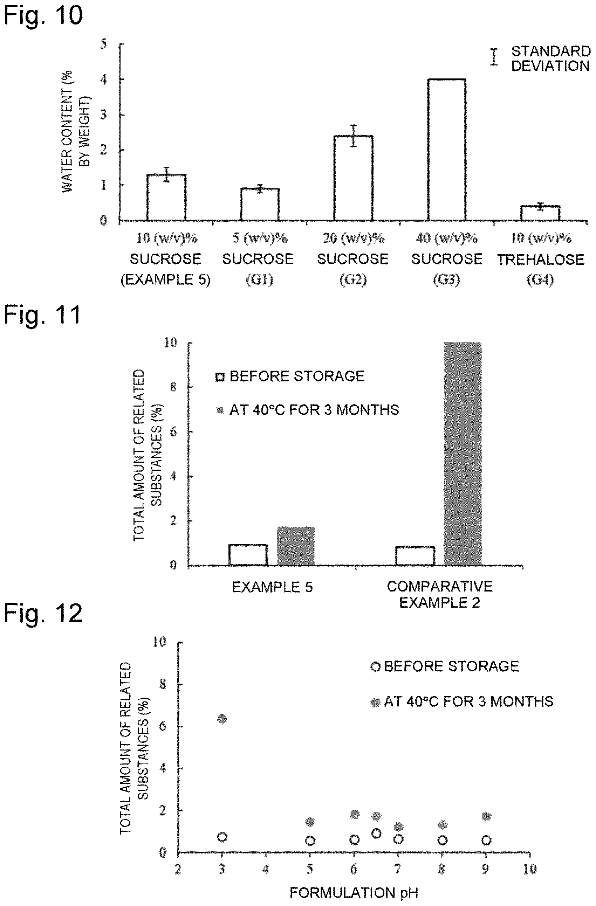

The evaluation results of the total amount of related substances for the storage stability obtained in Examples 5 to 8 and Comparative Example 2 are shown in Figs. 11 to 14.

### [Test Example 8]

A storage stability test (stored at 40°C and 75% RH for 3 months) was conducted on each sample to evaluate the stability of pharmaceutical compositions (lyophilized formulations). The cake height before and after storage was then evaluated. The measurement was performed by measuring the height from the highest position of the lyophilized cake to the bottom of the vial.

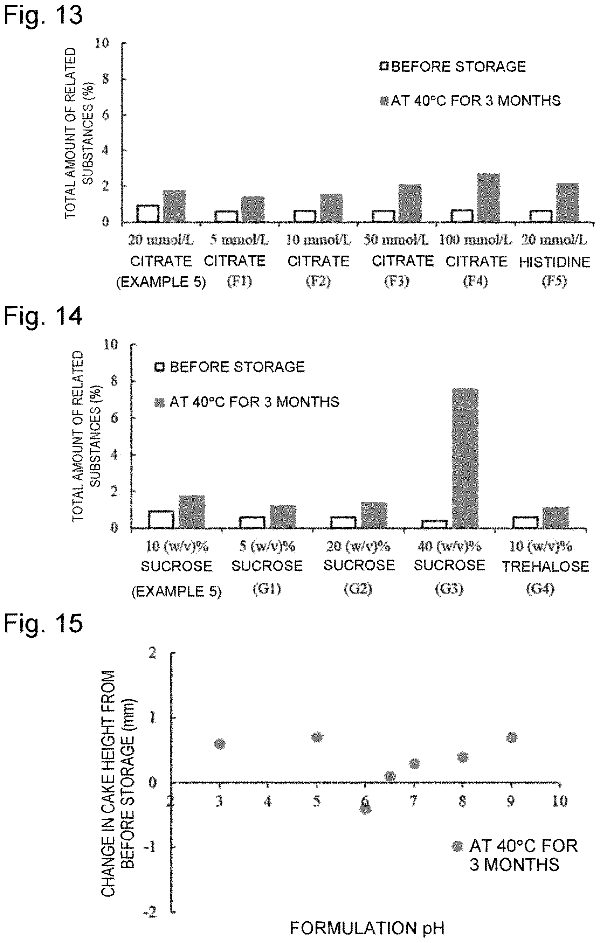

The evaluation results of the lyophilized cake height for the storage stability obtained in Examples 5 to 8 are shown in Figs. 15 to 17.

### [Test Example 9]

A storage stability test (stored at 40°C and 75% RH for 3 months) was conducted on each sample to evaluate the stability of pharmaceutical compositions (lyophilized formulations). The reconstitution time before and after storage was then evaluated. The test method is as follows.

Water was added into a vial with a syringe and an injection needle, and the time was measured using a time point after completion of the addition as the starting point. The mixture was reversed and mixed once per 5 seconds, and the presence or absence of the remaining from the dissolution was confirmed under a fluorescent light. The time point when particles were completely disappeared was recorded as the end point.

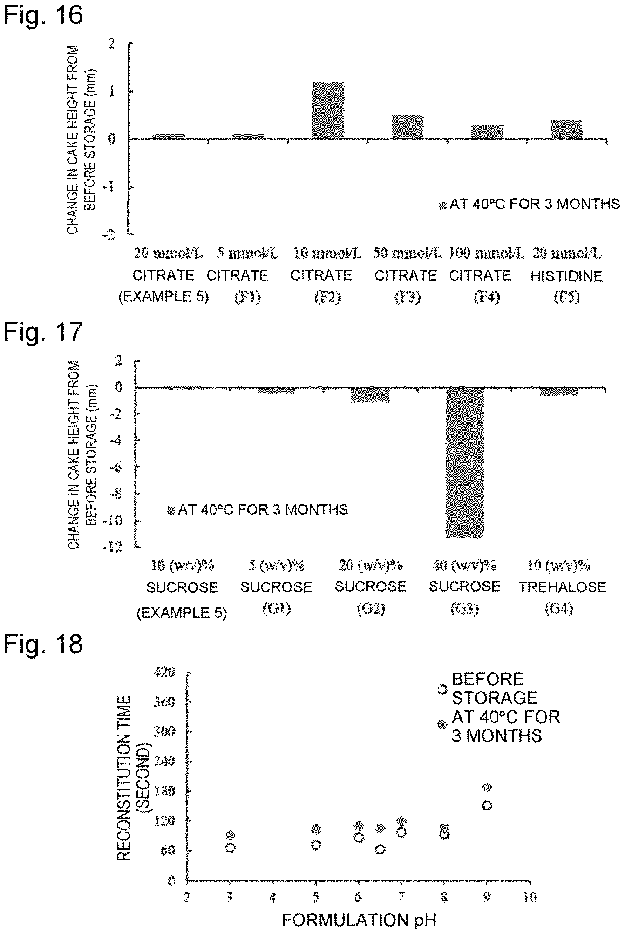

The evaluation results of the reconstitution time for the storage stability obtained in Examples 5 to 8 are shown in Figs. 18 to 20.

Fig. 11 can confirm that the storage stability was significantly improved by lyophilization.

Fig. 8 indicates that the water content of the lyophilized formulation before storage was around 1% by weight irrespective of pH, and equivalent among the samples.

Fig. 12 confirms that the amount of related substances significantly increased at pH 3 for the storage at 40°C and 75% RH for 3 months. On the other hand, the amount of related substances increased at pH 5 to 9 from before storage, but the stability was good. Fig. 15 indicates that no shrinkage of the lyophilized cake after storage was observed at any pH. Fig. 18 indicates that good re-solubility was exhibited at pH 3 to 8 before and after storage; however, it took time forreconstitution at pH 9 even before storage. Since the lyophilized formulation is redissolved at the time of use, around pH 6 to 7 is confirmed to be the optimal pH, grounded as well on the stability results of the liquids in Reference Examples 1 to 6.

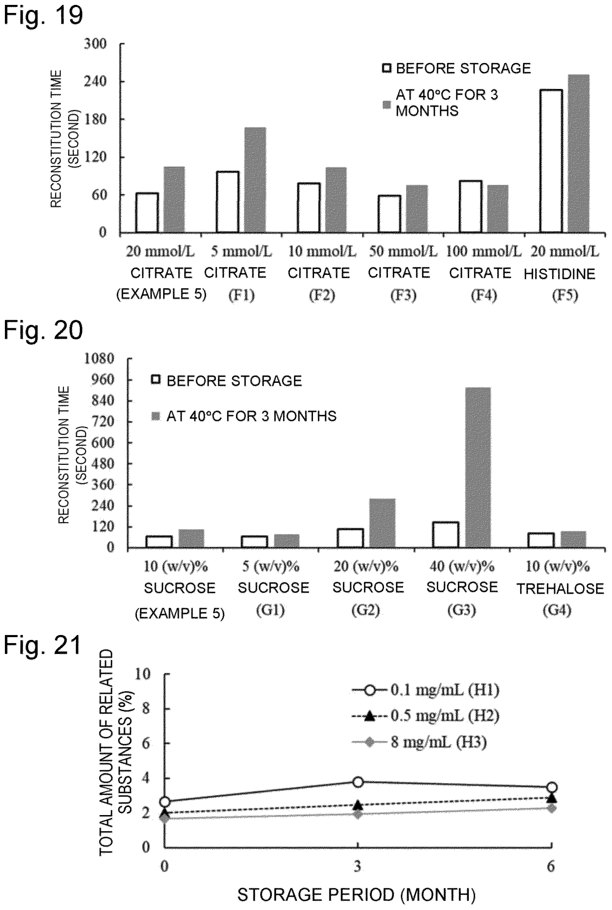

Fig. 9 indicates that the higher the concentration of the citrate buffer solution, the more the water content increases. Fig. 13 confirms that even though the higher the concentration of the citrate buffer solution, the more the amount of related substances increases after storage, the concentration range of 5 to 100 mmol/L satisfied the stability. For the histidine buffer solution, the stability under the storage conditions of 40°C and 75% RH was comparable to that of citrate buffer solution. Fig. 16 indicates that no shrinkage of the lyophilized cake after storage was observed at any concentration of citrate buffer solution nor histidine buffer solution. Fig. 19 confirms that the reconstitution time was extended in the cases of 5 mmol/L of citrate buffer solution after storage and the formulation of histidine buffer solution before and after storage. On the other hand, good re-solubility was exhibited in the formulation of 10 to 100 mmol/L of citrate buffer solution before and after storage. From the above results, it was confirmed that a stable lyophilized formulation can be obtained in the formulation of 10 to 100 mmol/L of citrate buffer solution, pH 6.5 containing 10% (w/v) sucrose.

Fig. 10 indicates that the water content significantly increased with the increase in the sucrose concentration, and when the sucrose concentration was 40% (w/v), the water content before storage was as high as about 4% by weight. Fig. 14 indicates that when the sucrose concentration was 5 to 20% (w/v), the stability was kept even after storage from the viewpoint of the related substances. On the other hand, in the formulation of 40% (w/v) sucrose, the total amount of related substances significantly increased after storage. This is largely caused by high water content before storage. When the excipient was 10% (w/v) trehalose, the storage stability was good from the viewpoint of the related substances. Fig. 17 indicates that the shape of the lyophilized cake was not able to be kept and was dissolved due to the storage in the formulation of 40% (w/v) sucrose. On the other hand, no significant shrinkage of the lyophilized cake before and after storage was observed when the sucrose concentration was 5 to 20% (w/v), and in the case of 10% (w/v) trehalose. Fig. 20 confirms that the reconstitution time was significantly extended in the formulation of 40% (w/v) sucrose associated with the dissolution of the lyophilized cake, and also, there was a tendency of extending the reconstitution time after storage in the formulation of 20% (w/v) sucrose. From the above results, it was confirmed that a stable lyophilized formulation can be obtained in the formulation of 20 mmol/L of citrate buffer solution containing 5 to 10% (w/v) sucrose or trehalose.

### [Example 9]

The same method as in Example 5 was used to prepare evaluation samples of sample Nos. H1 to H3, except that the pudexacianinium concentrations were set to 0.1, 0.5, and 8 mg/mL. The formulation of each evaluation sample is as shown in Table 13 below. The water content of the obtained pharmaceutical compositions (lyophilized formulations) were 1.2 to 1.4% by weight. The water content measurement is as shown in Test Example 10.

**[Table 13]**

| Sample No. | Pudexacianinium concentration | Buffer | pH | Excipient |
|---|---|---|---|---|
| H1 | 0.1 mg/mL | 20 mmol/L of citrate buffer solution | 6.5 | 10% (w/v) of sucrose |
| H2 | 0.5 mg/mL | | | |
| H3 | 8 mg/mL | | | |

### [Test Example 10]

A moisture analyzer (Arizona Instrument LLC or AMETEK Brookfield) was used to measure the water content of the lyophilized formulations in Example 9. The test method is as follows.

The water content was measured by measuring the weight of the vial containing the lyophilized cake, placing the sample in the moisture analyzer, and heating it. For the samples after the measurement, the weight of the lyophilized cake was calculated by washing and drying the vial, and measuring the weight of the empty vial. The water content was divided by the weight of the lyophilized cake to calculate the water content.

### [Test Example 11]

A storage stability test (stored at 40°C and 75% RH for 6 months) was conducted on each sample to evaluate the stability of pharmaceutical compositions (lyophilized formulations). The amounts of related substances of pudexacianinium before and after storage were then evaluated by a high performance liquid chromatography (HPLC). The test method and analysis conditions are as follows.

The samples were redissolved with water to have pudexacianinium concentration of 0.1 to 8 mg/mL, a mix solution of 10 mmol/L of phosphate buffer solution, pH 3.0/methanol (4:1) was added thereto to 0.1 mg/mL, and 100 µL thereof was injected. Except that, the same procedure was conducted as in Test Example 4

Peak areas of the related substances and pudexacianinium detected by HPLC were measured by an automated analysis method, and the peak area ratios (%) of each related substance and pudexacianinium were determined. The total amount of related substances was calculated by subtracting the peak area ratio of the detected pudexacianinium from 100%.

The evaluation results of the storage stability obtained in this Example are shown in Fig. 21. When the pudexacianinium concentration was set to 0.1 to 8 mg/mL, the increased amount of related substances during the storage was small in every case, and it was more stable with higher concentration. From the above results, it was confirmed that a stable lyophilized formulation can be obtained in the formulation of 20 mmol/L of citrate buffer solution, pH 6.5 containing 10% (w/v) sucrose at the pudexacianinium concentration of 0.1 to 8 mg/mL.

### [Example 10]

The same method as in Example 5 was used to prepare evaluation samples of sample Nos. I1 and I2, except that the pudexacianinium concentration were set to 2 and8 mg/mL. The formulation of each evaluation sample is as shown in Table 14 below. The water content of both the obtained pharmaceutical compositions (lyophilized formulations) was 2.5% by weight and 2.2% by weight, respectively. The water content measurement was conducted as shown in Test Example 10.

**[Table 14]**

| Sample No. | Pudexacianinium concentration | Buffer | pH | Excipient |
|---|---|---|---|---|
| I1 | 2 mg/mL | 20 mmol/L of citrate buffer solution | 6.5 | 10% (w/v) of sucrose |
| I2 | 8 mg/mL | | | |

### [Test Example 12]

A storage stability test (stored at 40°C and 75% RH for 6 months) was conducted on each sample to evaluate the stability of pharmaceutical compositions (lyophilized formulations). The amounts of related substances of pudexacianinium before and after storage were then evaluated by a high performance liquid chromatography (HPLC). The test method and analysis conditions are as follows.

The samples were redissolved with water to have a pudexacianinium concentration of 2 or 8 mg/mL. After that, the dilution and analysis were conducted in the same manner as in Test Example 10.

Peak areas of the related substances and pudexacianinium detected by HPLC were measured by an automated analysis method, and the peak area ratios (%) of each related substance and pudexacianinium were determined. The total amount of related substances was calculated by subtracting the peak area ratio of the detected pudexacianinium from 100%.

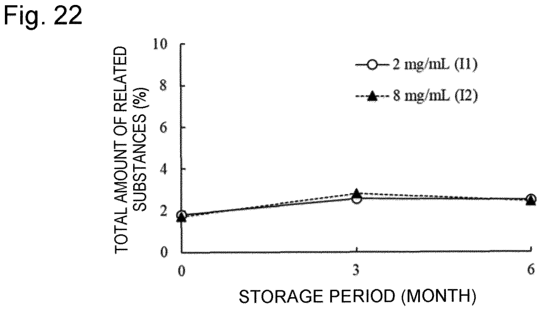

The evaluation results of the storage stability obtained in this Example are shown in Fig. 22. When the pudexacianinium concentration was set to 2 or 8 mg/mL, the increased amount of related substances during the storage was small in every case, and it was stable. From the above results, it was confirmed that a stable lyophilized formulation can be obtained in the formulation of 20 mmol/L of citrate buffer solution, pH 6.5 containing 10% (w/v) sucrose at the pudexacianinium concentration of 2 to 8 mg/mL.

### [Example 11]

A pudexacianinium pharmaceutical composition (lyophilized formulation) having water content of 1.1% by weight was prepared in the same method as in Example 5, except that the pudexacianinium concentration was set to 3 mg/mL. The water content measurement was conducted as shown in Test Example 10.

### INDUSTRIAL APPLICABILITY

The solid pharmaceutical composition of the present invention can be used in the field of near-infrared fluorescent imaging, for example.

## Claims

1. A solid pharmaceutical composition comprising pudexacianinium or a pharmaceutically acceptable salt thereof, a buffer, and an excipient, wherein water content in the solid pharmaceutical composition is 4% by weight or less.

2. The solid pharmaceutical composition according to claim 1, wherein the solid pharmaceutical composition in a form of an aqueous solution has a pH of 6 or more and 7 or less.

3. The solid pharmaceutical composition according to claim 1 or 2, wherein the buffer is selected from the group consisting of citrate, phosphate and histidine.

4. The solid pharmaceutical composition according to any one of claims 1 to 3, wherein a buffer concentration is 10 to 50 mmol/L when the solid pharmaceutical composition is in the form of an aqueous solution.

5. The solid pharmaceutical composition according to any one of claims 1 to 4, wherein the excipient is a sugar and/or a salt.

6. The solid pharmaceutical composition according to claim 5, wherein the sugar is sucrose and/or trehalose.

7. The solid pharmaceutical composition according to claim 6, wherein a concentration of sucrose and/or trehalose is 5 to 20% (w/v) when the solid pharmaceutical composition is in the form of an aqueous solution.

8. The solid pharmaceutical composition according to claim 5, wherein the salt is sodium chloride.

9. The solid pharmaceutical composition according to any one of claims 1 to 8, wherein a total amount of related substances after storage under stability test conditions of 40°C and a relative humidity of 75% for 1 month is 7.5% or less.

10. The solid pharmaceutical composition according to any one of claims 1 to 9, wherein the solid pharmaceutical composition is a lyophilized formulation.

11. The solid pharmaceutical composition according to any one of claims 1 to 10, wherein pudexacianinium or a pharmaceutically acceptable salt thereof is pudexacianinium chloride.

12. A method for producing the solid pharmaceutical composition according to claim 10, comprising (a) preparing a solvent solution containing a solvent, pudexacianinium or a pharmaceutically acceptable salt thereof, a buffer, and an excipient and adjusting a pH of the solvent solution, and (b) lyophilizing the solvent solution with the adjusted pH to obtain a lyophilizate.

13. A solid pharmaceutical composition comprising pudexacianinium or a pharmaceutically acceptable salt thereof, citric acid, and sucrose, wherein water content in the solid pharmaceutical composition is 4% by weight or less, and the solid pharmaceutical composition in a form of an aqueous solution has a pH of 6 or more and 7 or less.
